# EUROPEAN PATENT APPLICATION

(11) **EP 4 756 466 A2**
(43) Date of publication of application: **10.06.2026**
(21) Application number: 25215727.6
(22) Date of filing: 13.11.2025
(51) Int. Cl.: G01R 33/07, G01R 33/00

(54) **MAGNETIC SENSING DEVICE**

(30) Priority: 03.12.2024 IN 202411095090
(71) Applicant: Honeywell International Inc., Charlotte, NC 28202 (US)
(72) Inventor: SUYAMBU, Prathap, Charlotte, 28202 (US); NARAYANSWAMY, Nithin Mavinahaklu, Charlotte, 28202 (US); SAJJAN, Murgesh Ramappa, Charlotte, 28202 (US); NAIR, Ranjith Ramachandran, Charlotte, 28202 (US)
(74) Representative: Haseltine Lake Kempner LLP

(57) **Abstract**

Apparatuses, systems, and methods are provided for a magnetic sensing device comprising one or more linear magnetic sensors, such as Hall sensors. In some embodiments, the magnetic sensing device comprises a housing, a substantially cylindrical shield mechanically coupled to the housing, a magnet disposed within a cavity defined by the substantially cylindrical shield, wherein the magnet defines a countersunk cavity, and/or a printed circuit board assembly (PCBA) disposed proximate to a first end of the magnet that is opposite a second end of the magnet, wherein the second end of the magnet is proximate to an end of the housing proximate to a medium being measured.

## Description

### FIELD OF THE INVENTION

Embodiments of the present disclosure generally relate to magnetic sensing devices, particularly sensing devices comprising Hall sensors.

### BACKGROUND

Some sensing devices, in some examples, rely on passive magnets for sensing and/or attracting other materials. For example, some oil debris plugs comprise a plurality of stacked passive magnets configured to accumulate debris along the length of the oil debris plugs. Applicant has identified many technical challenges and difficulties associated with such passive sensors. Through applied effort, ingenuity, and innovation, many of these identified problems have been solved by developing solutions that are included in embodiments of the present disclosure, many examples of which are described in detail herein.

### BRIEF SUMMARY

Various example embodiments described herein relate to sensing devices, particularly sensing devices comprising Hall sensors.

In accordance with various embodiments of the present disclosure, an apparatus is provided comprising: (i) a housing; (ii) a substantially cylindrical shield mechanically coupled to the housing; (iii) a magnet disposed within a cavity defined by the substantially cylindrical shield, wherein the magnet defines a countersunk cavity; and (iv) a printed circuit board assembly (PCBA) disposed proximate to a first end of the magnet that is opposite a second end of the magnet, wherein the second end of the magnet is proximate to an end of the housing proximate to a medium being measured.

In some embodiments, the PCBA comprises a Hall sensor protruding from a surface of the PCBA, and wherein the protruding Hall sensor is disposed within the countersunk cavity defined by the magnet.

In some embodiments, the substantially cylindrical shield is configured to allow a change in magnetic flux sensed by the Hall sensor to be substantially uniformly sensed by the Hall sensor.

In some embodiments, the magnet is axially magnetized with respect to the countersunk hole, and wherein the Hall sensor is positioned at an angle to an axis of magnetization of the magnet.

In some embodiments, the magnet is configured to generate a zone of approximately zero magnetic field strength in which the Hall sensor is disposed.

In some embodiments, the zone of approximately zero magnetic field strength is configured to allow substantially linear operation of the Hall sensor.

In some embodiments, the apparatus is configured to identify rapid changes in Hall voltage arising from rapid changes in debris presence on at least one surface of the housing.

In accordance with various embodiments of the present disclosure, a system is provided comprising: (1) a fluid container; and (2) a magnetic sensing device for sensing debris in a fluid of the fluid container, the magnetic sensing device comprising: (i) a housing; (ii) a substantially cylindrical shield mechanically coupled to the housing; (iii) a magnet disposed within a cavity defined by the substantially cylindrical shield, wherein the magnet defines a countersunk cavity; and (iv) a printed circuit board assembly (PCBA) disposed proximate to a first end of the magnet that is opposite a second end of the magnet, wherein the second end of the magnet is proximate to an end of the housing proximate to a medium being measured.

In some embodiments, the PCBA comprises a Hall sensor protruding from a surface of the PCBA, and wherein the protruding Hall sensor is disposed within the countersunk cavity defined by the magnet.

In some embodiments, the substantially cylindrical shield is configured to allow a change in magnetic flux sensed by the Hall sensor to be substantially uniformly sensed by the Hall sensor.

In some embodiments, the magnet is axially magnetized with respect to the countersunk hole, and wherein the Hall sensor is positioned at an angle to an axis of magnetization of the magnet.

In some embodiments, the magnet is configured to generate a zone of approximately zero magnetic field strength in which the Hall sensor is disposed.

In some embodiments, the zone of approximately zero magnetic field strength is configured to allow substantially linear operation of the Hall sensor.

In some embodiments, the sensing device is configured to identify rapid changes in Hall voltage arising from rapid changes in debris presence on at least one surface of the housing.

In accordance with various embodiments of the present disclosure, a method is provided comprising: (i) mechanically coupling a substantially cylindrical shield to a housing; (ii) disposing a magnet within a cavity defined by the substantially cylindrical shield, wherein the magnet defines a countersunk cavity; and (iii) disposing a printed circuit board assembly (PCBA) proximate to a first end of the magnet that is opposite a second end of the magnet, wherein the second end of the magnet is proximate to an end of the housing proximate to a medium being measured.

In some embodiments, the PCBA comprises a Hall sensor protruding from a surface of the PCBA, and wherein the protruding Hall sensor is disposed within the countersunk cavity defined by the magnet.

In some embodiments, the method further comprises magnetizing the magnet axially with respect to the countersunk hole such that the Hall sensor is positioned at an angle to an axis of magnetization of the magnet.

In some embodiments, the method further comprises configuring the magnet to generate a zone of approximately zero magnetic field strength in which the Hall sensor is disposed.

In some embodiments, the method further comprises configuring the zone of approximately zero magnetic field strength to allow substantially linear operation of the Hall sensor.

In some embodiments, the method further comprises configuring an apparatus implementing the method to identify rapid changes in Hall voltage arising from rapid changes in debris presence on at least one surface of the housing.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a cross-sectional view of an example magnetic sensing device comprising a Hall sensor;
FIG. 2A is a top-down view of at least a portion of the magnetic sensing device of FIG. 1;
FIG. 2B is a cross-sectional view of at least a portion of the magnetic sensing device of FIG. 1; and
FIG. 3 is a flowchart of an exemplary method of constructing a magnetic sensing device comprising a Hall sensor, in accordance with some embodiments of the present disclosure.

### DETAILED DESCRIPTION OF THE INVENTION

### Introduction

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically coupled," "electrically coupling," "electrically couple," "electrically connected," "electrically connecting," "electrically connect," "in communication with," or "in electronic communication with" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "sensor" or "sensing device" refers to a component or device that may detect, measure, and/or identify any one or more attributes or characteristics of an environment of media, including but not limited to presence of other media, proximity of other media and/or objects, and/or the like.

### Overview

In some examples, sensing devices, such as magnetic sensing devices, may be configured to monitor fluids of various types of systems (e.g., such as engines, transmissions, gearboxes, radiators, cooling systems, and/or other systems) for presence of debris (e.g., metal or magnetic debris) which may originate from various components of the systems (e.g., bearings, gears, and/or the like). In some examples, such a sensing device may be an oil debris plug configured to accumulate metal debris present in oil of a vehicle, for example. In some examples, the oil debris plug may comprise a plurality of stacked passive magnets configured to accumulate debris along the length of the oil debris plug. Such a configuration, however, may not allow for "face-sensing", which is a term that refers to sensing performed at an end and/or surface (e.g., a face) of a sensing device.

Embodiments of the present disclosure, in some examples, provide a magnetic sensing device. Example embodiments of the present disclosure, in some examples, may include a magnetic sensing device configured to measure presence and/or proximity at a first end or surface of the magnetic sensing device. Such a magnetic sensing device may include any type of linear magnetic sensor, for example, such as Hall sensors, anisotropic magnetoresistive (AMR) sensors, giant magnetoresistance (GMR) sensors, tunnel magnetoresistance (TMR) sensors, and/or the like. A magnetic sensing device may comprise one or more Hall sensors. For example, a magnetic sensing device may comprise a housing, such as a housing configured to at least partially surround one or more components of the magnetic sensing device. The magnetic sensing device may further comprise a substantially cylindrical shield mechanically coupled to the housing, such as via adhesive and/or snapping together. The magnetic sensing device may further comprise a magnet disposed within a cavity defined by the substantially cylindrical shield. For example, the magnet may define a cavity, such as a countersunk cavity. The cavity defined by the magnet may be substantially cylindrical and/or comprised of another geometry. The magnetic sensing device may further comprise a printed circuit board assembly (PCBA) disposed proximate to a first end of the magnet that is opposite a second end of the magnet, wherein the second end of the magnet is proximate to an end of the housing proximate to a medium being measured.

Embodiments of the present disclosure, in some examples, allow for a sensing device to perform face-sensing. In some examples, face-sensing further comprises sensing, by an end and/or surface of a housing proximate to a medium being measured, one or more properties of the medium and/or its environment. For example, the magnetic sensing device may be configured to sense, via one or more Hall sensors comprised by the magnetic sensing device, proximity of one or more objects to the end of the magnetic sensing device and/or an amount (or quantity) of debris at the end of the magnetic sensing device while allowing at least one magnet, comprised by the magnetic sensing device, to accumulate the debris along the sides of the housing. The magnet may define a substantially cylindrical cavity at approximately its center and may be at least partially (e.g., fully) surrounded by a substantially cylindrical shield. The magnet may generate a magnetic field defining a "null zone" of approximately zero magnetic field strength in the substantially cylindrical cavity. A PCBA comprising a Hall sensor may be disposed proximate to a first end of the magnet such that the Hall sensor protrudes into the substantially cylindrical cavity and, thus, into the null zone. Positioning of the Hall sensor within the null zone may allow for linear operation of the Hall sensor. A second end of the magnet may be disposed proximate to the end of the housing proximate to the medium being sensed, increasing magnetic field strength at the "head" of the magnetic sensing device (e.g., an end proximate to and/or protruding into the medium being measured).

As described herein, embodiments of the present disclosure, in some examples, provide apparatuses, systems, and/or methods for sensing devices, such as sensing devices comprising Hall sensors.

To address challenges and limitations associated with sensing devices, various examples of the present disclosure may be provided. For example, various examples of the present disclosure may provide example apparatuses, systems, and/or methods for magnetic sensing devices, such as sensing devices comprising one or more Hall sensors.

### Exemplary Apparatuses, Systems, and Methods

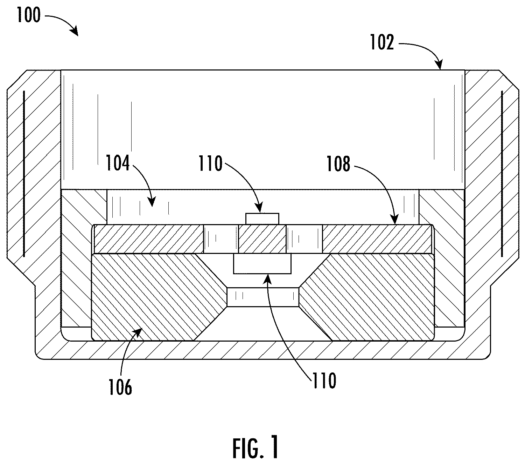

Referring now to FIG. 1, a cross-sectional view of an example magnetic sensing device 100 comprising a Hall sensor is provided. In the example of FIG. 1, the magnetic sensing device 100 comprises a housing 102, a shield 104, a magnet 106, a printed circuit board assembly (PCBA) 108, and a Hall sensor 110. Although the example of FIG. 1 shows one housing, one shield, one magnet, one PCBA, and one Hall sensor, any number of these components may be present in a magnetic sensing device 100.

The housing 102 may be comprised of plastic and/or other materials. The housing 102 being comprised of plastic may yield some advantages including, for example, not interfering with magnetic measurements performed by the magnetic sensing device 100. In some examples, the housing 102 is configured to at least partially surround one or more components of the magnetic sensing device 100. For example, the housing 102 may be configured to surround the shield 104, the magnet 106, the PCBA 108, and the Hall sensor(s) 110. The housing 102 may be further configured to protect the one or more components of the magnetic sensing device 100 from contamination and/or debris originating from the material and/or medium being measured. For example, in an example where the magnetic sensing device 100 is an oil debris plug, the housing 102 may be configured to prevent oil from entering into and/or damaging internal portions of the magnetic sensing device 100.

The shield 104 may be comprised of metal and/or other materials. The shield 104 may be a substantially cylindrical shell. In some examples, the shield 104 may comprise a protruding feature which protrudes toward a center of the of the shield 104. The protruding feature may be configured to act as a mechanical stop, maintaining a position of one or more components of the magnetic sensing device 100. The shield 104 may at least partially surround the magnet 106, the PCBA 108, the Hall sensor 110, and/or other components of the magnetic sensing device 100. The shield 104 may be configured to adjust the null zone. For example, the shield 104 may be configured to extend the null zone beyond the magnet 106. For example, the null zone may be shifted, compared with a magnet having no shield, towards one or more ends of the magnet 106. The shield 104 may be configured to allow the Hall sensor 110 to experience substantially a total magnetic field strength and/or magnetic flux generated, for example, by ferrous debris accumulating along the length and/or at the head of the magnetic sensing device 100.

The magnet 106 may be a permanent magnet, an electromagnet, and/or another type of magnet. In some examples, the magnet 106 is a permanent magnet. The magnet 106 may define a cavity approximately at its center configured to at least partially contain the null zone. The cavity of the magnet 106 may be substantially cylindrical and/or comprised of other geometries. In some examples, the magnet 106 comprises a cavity of an approximately hourglass shape.

The PCBA 108 may comprise the Hall sensor 110 and/or other electronic components. For example, the Hall sensor 110 may be comprised of one or more portions, such as a first portion is electrically coupled to a "top" (e.g., first) surface of the PCBA 108 and a second portion electrically coupled to a "bottom" (e.g., second) surface of the PCBA 108. The top surface of the PCBA 108 may be opposite the bottom surface of the PCBA 108.

The Hall sensor 110 may be comprised of one or more components. For example, the Hall sensor 110 may be comprised of the first portion electrically coupled to the PCBA 108 and/or a second portion coupled to an opposite side of the PCBA 108. The Hall sensor 110 may be configured to convert received and/or measured magnetic field strength into electrical signals, for example, and the PCBA 108 may be configured to transmit the electrical signals to one or more devices and/or components thereof.

In some examples, the magnetic sensing device 100 is an oil debris plug, for example, configured to collect ferrous debris along a length of the magnetic sensing device 100. The magnet 106 may generate a null zone at approximately the center of the cavity defined by the magnet 106. The shield 104 of the magnetic sensing device 100 may be configured to extend the null zone beyond the magnet 106 such that the null zone is at least partially in a region at one or more ends of the magnet 106. For example, the shield 104 may be configured to enable a magnetic field generated by debris to be focused on a sensing end of the magnetic sensing device 100. In some examples, the shield 104 may be configured to allow debris accumulated on the magnetic sensing device 100 to generate a change in magnetic flux that can be sensed by the Hall sensor 110.

In some examples, the magnetic sensing device 100 is a proximity sensor, for example, configured to determine proximity of one or more objects (e.g., comprised of a magnetic material) to the magnetic sensing device 100. In a proximity sensor, the shield 104 may be configured to extend a sensing range of the proximity sensor by shifting a region defined by the null zone.

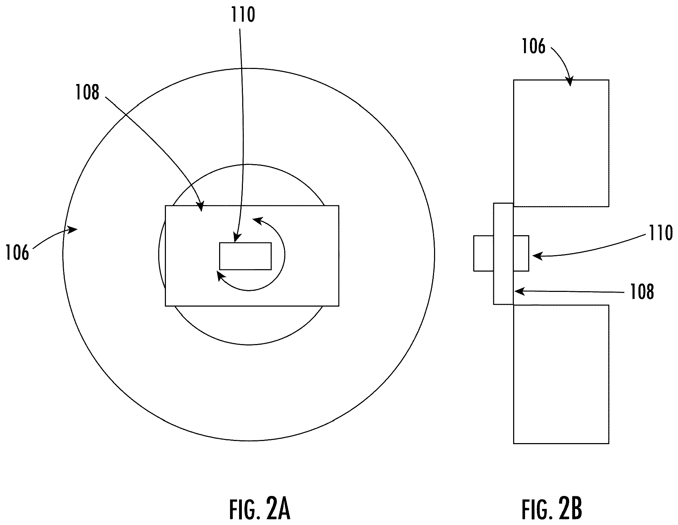

Referring now to FIGS. 2A-2B, top-down and cross-sectional view of the magnetic sensing device 100 are provided, respectively. In the example of FIG. 2A, the rounded double-ended arrow indicates that the Hall sensor 110 may be rotated about its axis such that it faces any direction, since the Hall sensor 110 responds substantially linearly to the axial component of the applied magnetic field. The axis about which the Hall sensor 110 rotates may be substantially perpendicular to a plane of the magnet 106. In the example of FIG. 2B, the reference numeral 110 indicates that the Hall sensor may protrude from either end of the PCBA 108. Furthermore, the Hall sensor 110 may be disposed in any one or more of the following regions: fully in the null zone, partially in the null zone, and/or near the null zone. In some embodiments, the Hall sensor 110 may be disposed near the null zone such that the applied magnetic field is not high enough to saturate the Hall sensor 110.

In at least some technologies, magnets configured to produce a first magnetic field strength may be relied upon to remove and/or attract debris from a medium into which the sensing device a (e.g., a fluid). To accommodate the first magnetic field strength, a Hall sensor with linear output ranging from approximately -300 mT to approximately 300 mT. A magnet defining a cavity at approximately its center may be configured to generate a null zone in the cavity. The Hall sensor may be coupled to a first surface of a PCBA, wherein one or more other electronic components may be coupled to the second surface of the PCBA, the second surface being opposite the first surface. The PCBA may be placed proximate to the magnet and enclosed by the shield such that the Hall sensor protrudes at least partially into the cavity of the magnet. In some examples, the geometry of the shield may allow for changes in magnetic flux arising from debris collected on an outer surface of the sensing device to be substantially uniformly experienced by the Hall sensor.

In some examples, the null zone may be adjusted based on design of the shield. For example, the Hall sensor may be disposed at least partially within the null zone, such that substantially linear operation of the Hall sensor may be relied upon. In some examples, axis-symmetry may be maintained (with respect to the sensing device), allowing magnetic field strength, magnetic flux, and/or other properties arising from debris deposition.

In some examples, debris accumulated at the "face" of the sensing device may create a low reluctance return path for magnetic flux, thus increasing the magnetic field through the Hall sensor. Furthermore, debris accumulated at the face may be sensed with greater sensitivity due to proximity to the Hall sensor.

In some examples, the magnet may be axially magnetized, such that magnetic field at an end of the sensing device is axis-symmetric. The Hall sensor may then be disposed at any angle ranging from 0 degrees to 360 degrees with respect to the axis of magnetization. Additionally or alternatively, the Hall sensor may be disposed proximate to any surface of the PCBA.

Some advantages of the embodiments described herein, in some examples, include monitoring real-time debris deposition (e.g., to avoid catastrophic failure(s) in operation). Such monitoring may be achieved via configuring a response of the Hall sensor with respect to at least one weight of the debris (e.g., using linearization algorithm, lookup table(s), and/or the like).

In some examples, an amount and/or quantity of deposited debris may range from approximately 3 grams to approximately 6 grams (e.g., 1-10 grams, preferably 3 to 6 grams). In such examples, the Hall sensor may be configured to respond with respect to a rate of deposition of the debris. For example, based on a rate of change of a Hall voltage, surge(s) in amount of debris (e.g., arising as a result of failure of one or more components of a device and/or system that comprises the sensing device) may be identified (e.g., via implemented one or more predetermined algorithms).

In some examples, output from the sensing device may be digital (e.g., SPI, SENT, I2C, etc.) or analog (e.g., Voltage, Current, PWM, etc.). Moreover, the sensing device may be coupled (e.g., via electrical connection and/or the like) to a microcontroller, for example, which is configured to output to a main system via digital (e.g., including CAN), analog, or wireless (BLE, Wi-Fi, etc.) communication.

In some examples, amount(s) and/or quantity(ies) of debris deposition may be varied by increasing or decreasing a quantity of magnets comprised by the sensing device. For example, the deposition of debris may be made more uniform by increasing one or more first gaps (e.g., via tuning thickness(es) of spacers between the magnets) between magnets. Additionally or alternatively, changing one or more second gaps between magnets and polepieces may increase uniformity of debris deposition. In some examples, debris deposition area(s) may be adjusted by adding spacers and/or adjusting polepieces and/or other components of the sensing device.

In some examples, the sensing device is configured to distinguish particle size of the debris, thereby having the capability to identify specific materials leading to failure of the overall device and/or system, as different materials have different permeabilities (e.g., which is the feature affecting changes in Hall voltage of the sensing device.

Further advantages of the embodiments described herein include: predicting failure(s) and avoiding downtime by distinguishing regular wear and/or case(s) of system breakdown.

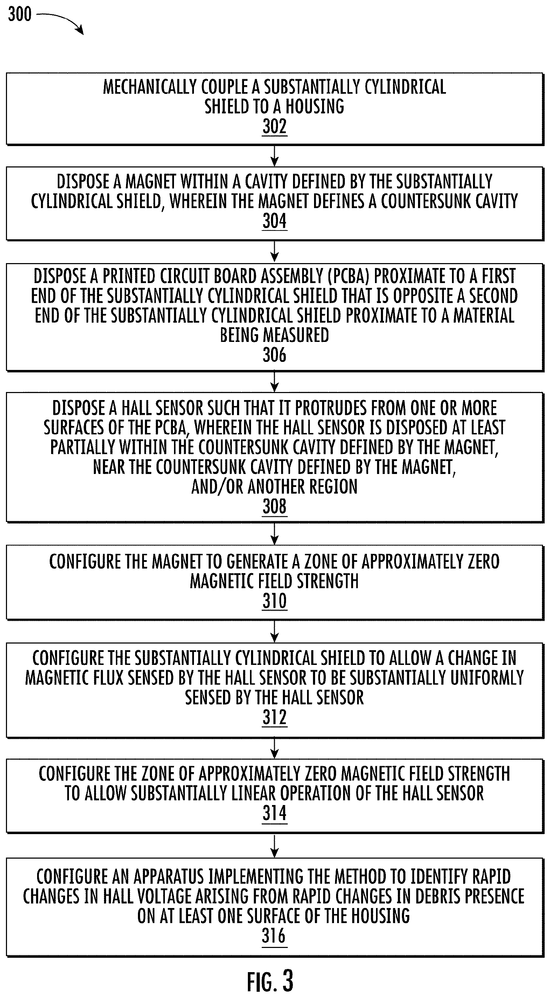

Referring now to FIG. 3, a flowchart of an exemplary method 300 of constructing a sensing device comprising a Hall sensor is provided.

At step/operation 302, a substantially cylindrical shield may be coupled to a housing.

At step/operation 304, a magnet may be disposed within a cavity defined by the substantially cylindrical shield, wherein the magnet defines a countersunk cavity.

At step/operation 306, a PCBA may be disposed proximate to a first end of the substantially cylindrical shield that is opposite a second end of the substantially cylindrical shield proximate to a material being measured.

At step/operation 308, a Hall sensor may be disposed such that it protrudes from one or more surfaces of the PCBA. The Hall sensor may be disposed at least partially within at least one of the following regions: the countersunk cavity defined by the magnet, near the countersunk cavity defined by the magnet, and/or another region.

At step/operation 310, the magnet may be configured to generate a zone of approximately zero magnetic field strength.

At step/operation 312, the substantially cylindrical shield may be configured to allow a change in magnetic flux sensed by the Hall sensor to be substantially uniformly sensed by the Hall sensor.

At step/operation 314, the zone of approximately zero magnetic field strength may be configured to allow substantially linear operation of the Hall sensor.

At step/operation 316, an apparatus implementing the method 300 may be configured to identify rapid changes in Hall voltage arising from rapid changes in debris presence on at least one surface of the housing.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

### Conclusion

While various embodiments in accordance with the principles disclosed herein have been shown and described /above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. An apparatus comprising:
a housing;
a substantially cylindrical shield mechanically coupled to the housing;
a magnet disposed within a cavity defined by the substantially cylindrical shield, wherein the magnet defines a countersunk cavity; and
a printed circuit board assembly (PCBA) disposed proximate to a first end of the magnet that is opposite a second end of the magnet, wherein the second end of the magnet is proximate to an end of the housing proximate to a medium being measured.

2. The apparatus of claim 1, wherein the PCBA comprises a Hall sensor protruding from a surface of the PCBA, and wherein the protruding Hall sensor is disposed at least partially within at least one of the following regions:
the countersunk cavity defined by the magnet; or
near the countersunk cavity defined by the magnet.

3. The apparatus of claim 2, wherein the magnet is axially magnetized with respect to the countersunk hole, and wherein the Hall sensor is rotated about an axis of magnetization of the magnet.

4. The apparatus of claims 2 or 3, wherein the magnet is configured to generate a zone of approximately zero magnetic field strength.

5. The apparatus of claim 4, wherein the substantially cylindrical shield is configured to allow a change in magnetic flux sensed by the Hall sensor to be substantially uniformly sensed by the Hall sensor.

6. The apparatus of claim 5, wherein the zone of approximately zero magnetic field strength is configured to allow substantially linear operation of the Hall sensor.

7. The apparatus of claims 2-6, wherein the apparatus is configured to identify rapid changes in Hall voltage arising from rapid changes in debris presence on at least one surface of the housing.

8. A method comprising:
mechanically coupling a substantially cylindrical shield to a housing;
disposing a magnet within a cavity defined by the substantially cylindrical shield, wherein the magnet defines a countersunk cavity; and
disposing a printed circuit board assembly (PCBA) proximate to a first end of the magnet that is opposite a second end of the magnet, wherein the second end of the magnet is proximate to an end of the housing proximate to a medium being measured.

9. The method of claim 8, wherein the PCBA comprises a Hall sensor protruding from a surface of the PCBA, and wherein the protruding Hall sensor is disposed at least partially within at least one of the following regions:
the countersunk cavity defined by the magnet; or
near the countersunk cavity defined by the magnet.

10. The method of claims 8 or 9, further comprising:
configuring the magnet to generate a zone of approximately zero magnetic field strength.

11. The method of claim 10, further comprising:
configuring the substantially cylindrical shield to allow a change in magnetic flux sensed by the Hall sensor to be substantially uniformly sensed by the Hall sensor.

12. The method of claims 11 or 12, further comprising:
configuring the zone of approximately zero magnetic field strength to allow substantially linear operation of the Hall sensor.

13. The method of claims 9-12, further comprising:
configuring an apparatus implementing the method to identify rapid changes in Hall voltage arising from rapid changes in debris presence on at least one surface of the housing.
